# EUROPEAN PATENT APPLICATION

(11) **EP 4 099 332 A1**
(43) Date of publication of application: **07.12.2022**
(21) Application number: 21177157.1
(22) Date of filing: 01.06.2021
(51) Int. Cl.: G16B 40/10, G16B 45/00

(54) **METHOD FOR REVIEWING AND MANIPULATING OF DIGITAL PCR DATA**

(71) Applicant: QIAGEN GmbH, 40724 Hilden (DE)
(72) Inventor: WOZNIK, Maria, 40724 Hilden (DE); MATTHAEI, Kevin, 40724 Hilden (DE); WROBLEWSKI, Mateusz, 40724 Hilden (DE)
(74) Representative: König Szynka Tilmann von Renesse Patentanwälte Partnerschaft mbB Düsseldorf

(57) **Abstract**

The present invention provides methods for visualization, reviewing and manipulating of dPCR data. Furthermore, the present invention provides systems for dPCR data visualization, reviewing and manipulating and computer-readable storage media with instructions for dPCR data visualization, reviewing and manipulating. In addition the use of such systems and computer-readable storage media for dPCR data visualization, reviewing and manipulating is provided.

## Description

### FIELD OF THE INVENTION

The present invention provides improved methods for visualization, reviewing and manipulating of data obtained by a biological analysis system, such as digital polymerase chain reaction (dPCR). Furthermore, the present invention provides a system and a computer-readable storage medium for reviewing and manipulating of data obtained by a biological analysis system.

### BACKGROUND OF THE INVENTION

Methods for visualization of data obtained by biological analysis systems, which are able to process a high number of samples simultaneously, is of high relevance since the immense number of data points needs to be displayed to the user in a useful way. Such biological analysis systems are commonly used in polymerase chain reaction (PCR), sequencing, genotyping and other applications to provide quantitative data.

One technology that has gained increasing importance in recent years is digital PCR (dPCR). In dPCR, each sample containing a small number of target nucleic acids is divided into lots of subsamples, also called partitions. Consequently, each partition either contains the target nucleic acid or not. Subsequently, an end-point PCR according to standard protocols is executed and a signal for each partition is measured in a certain position. For instance, for a partition containing the target nucleic acid a positive detection signal may be measured due to an amplification reaction while for a partition that does not contain the target nucleic acid no detection signal is generated since no amplification reaction occurred. Thus, dPCR uses the standard procedure of end-time PCR, but splits the PCR in many single partitions in which the target is randomly distributed across all available partitions. As the target is distributed randomly, the amount of target nucleic acid per positive partition can be calculated using Poisson statistics. Such dPCR systems combine high-throughput screening and multiplexing capabilities with high sensitivity, excellent precision and very good reproducibility. Several dPCR systems are commercially available, e.g. the QIAcuity systems (QIAGEN, Hilden), which are designed for applications as rare mutation detection, copy number variation, gene expression analysis, NGS (next generation sequencing) library quantification, (low-level) pathogen detection, viral load detection, genotyping, miRNA research, IncRNA analysis, and genetically modified organisms (GMO) detection.

The process of partitioning, thermocycling, imaging (i.e. data collection), and analysis is commonly implemented in a biological analysis system, such as the QIAcuity instrument (QIAGEN, Hilden). These systems at least include a thermal cycler, an optical system, one or more processors, a memory, a storage device, and a display. In dPCR, the signals are usually fluorescence signals emitted from fluorophores and detected in different channels according to the wavelength of the emitted signals. Typically, one or more fluorophores are used. Based on all signals detected in one channel, a threshold can be calculated to distinguish between positive and negative partitions in that channel, corresponding to the partitions where an amplification reaction occurred or not. As a result, these systems provide the concentration in copies per microliter of the target nucleic acids as well as quality control such as positive samples or non-template controls (NTC).

Due to the high number of amplification reactions performed simultaneously in dPCR applications, a huge amount of data is obtained that needs to be further processed and visualized to allow data interpretation by the user. Commonly, the processed data are displayed to the user at a display via a graphical user interface (GUI). Different methods for dPCR data visualization have been disclosed by the prior art.

For example, EP 3014506 A1 teaches a method for visualization of dPCR data by displaying the relative location (x, y coordinates) where the underlying signal was detected on a chip.

Furthermore, the data may be displayed in a histogram that visualizes the distribution of fluorescence intensity values for a selected target channel, e.g. FAM. The x-axis represents the fluorescence intensity and the y-axis the number of partitions having that fluorescence intensity. Typically, partitions where an amplification reaction occurred will have a high fluorescence intensity while partitions where no amplification reaction can be detected will have a low fluorescence intensity resulting in two distinct peaks.

Moreover, the data may be visualized as a scatter plot. For example, the two-dimensional (2D) scatter plot shows the fluorescence intensity from two selected channels by plotting the fluorescence intensity of one channel against the fluorescence intensity of the other channel. Partitions with no amplification reaction (negative) occur as a cluster having low fluorescence intensities in both channels while partitions where a signal was detected (positive) are clustered according to the respective dye used for target nucleic acid labelling. For instance, partitions for which a signal could be detected in FAM channel appear as one cluster, partitions for which a signal could be detected in Cy5 channel appear as a second cluster, partitions for which signals could be detected in both channels appear as a third cluster, and partitions for which no signal could be detected neither in FAM nor in Cy5 channel (negative) appear as a fourth cluster. Such representations of two-dimensional scatter plots are described in US 2015/0269756 A1 and provided by the QIAcuity software (QIAGEN, Hilden).

Data visualization can be manipulated by the user by adjusting the signal intensity threshold for the distinction between positive and negative partitions. Selection of a certain threshold changes the visualization of the data accordingly.

Furthermore, in some partitions a positive signal may be detected that differs from the expected signal for a successful amplification (false positive) or any other erroneous event occurs leading to invalidity of those partitions. In such a case it is important to identify those events and to correct the data accordingly by excluding all partitions that have to be corrected from the analysis to allow for reliable data interpretation. Such methods for data correction by excluding invalid signals are provided in the QIAcuity software (QIAGEN, Hilden).

The specific characteristics of the signal from each partition lead to a lot of information that is available for the analyzed sample. This information has to be visualized in a useful way such that the quality of the data can be evaluated and the results adequately interpreted. Identification of partitions that have to be corrected is of particular importance in a situation in which precise quantification of minimal amounts of target nucleic acids or target nucleic acid detection in complex samples is required, such as wastewater testing for severe acute respiratory syndrome coronavirus type 2 (SARS-CoV-2) or identification of genetic disorders including the rarest of cancer mutations. In this regard, partitions that have to be corrected have a significant influence on data analysis and interpretation and subsequent measures. However, prior art methods only provide identification of erroneous events and subsequent data correction by exclusion of partitions that have to be corrected as described above but do not allow for reconsidering or visualization of the reason due to which a signal or partition was corrected. Therefore, until today no method for dPCR data visualization has been provided that allows for visualization by distinguishing between different categories of corrected signals or corrected partitions according to the reason for correction. Furthermore, prior art methods do not allow for manually excluding or including such categories from visualization and reclassification of members of different categories by the user.

### SUMMARY OF THE INVENTION

The present invention overcomes core drawbacks of the prior art. In particular, the present invention provides improved methods for reviewing and manipulating of data obtained by a biological assay, such as dPCR, performed in a biological analysis system that allow reliable results as basis for data interpretation. A signal map representing preprocessed or corrected data, respectively, i.e. the signal intensity for each valid or correctly assigned partition, respectively, is used as a basis for the method of the present invention.

In particular, useful methods are provided for processing of data determined by a biological analysis system, such as dPCR data, that allow reliable interpretation of the presence or absence of target molecules, such as nucleic acids, in the analyzed samples. The technology described herein is based on the classification of detected signals emitted from the reaction sites, termed partitions, in a region of a substrate containing the analyzed sample. Furthermore, the method disclosed herein provides the, preferable automatic, identification of signals that have to be corrected and the, preferable automatic, classification of those signals that have to be corrected according to the reason for correction. The reviewing method disclosed herein provides the automatic visualization of the data in a signal map representing the partitions located at a region of a substrate according to their spatial location in that region and according to the respective category or classification. In addition, the manipulation method provides manual adjustment of the representation of categories or classifications in the signal map by the user in response to manipulation of a first interactive tool. A second interactive tool allows the user to manually reclassify single partitions which had been automatically classified to a different category. Thereby, the method of the present invention particularly improves dPCR data processing and provides more reliable results on the presence or absence of target nucleic acids in a biological sample. The method described herein is in particular suitable for the detection of target nucleic acids in complex biological samples and/or in biological samples in which the target nucleic acids are only present in minimal quantities which regularly leads to erroneous measurements. Thus, the method provided herein combines the greater accuracy of dPCR compared to traditional real-time PCR and reliable data analysis also of error-prone samples. The present invention therefore makes an important contribution to the art.

According to a first aspect, a method for reviewing and manipulating of data obtained from signals determined by a biological analysis system is provided, comprising the steps:
(A) receiving a plurality of detected signals from a plurality of partitions located at a region of a substrate subjected to a biological assay;
(B) choosing a value for a signal intensity threshold;
(C) classifying the partitions for which a signal intensity is detected above the signal intensity threshold as positive and/or the partitions for which a signal intensity is detected below the signal intensity threshold as negative;
(D) identifying partitions that have to be corrected, so that some or all of the partitions that have to be corrected are classified as negative and/or as invalid, so that the partitions that have to be corrected are corrected partitions, and classifying all partitions classified as positive and negative as valid;
(E) displaying a graphical user interface comprising a signal map of the detected signals, wherein the signal map represents the valid partitions by their signal intensities, wherein the corrected negative partitions are represented according to the signal intensity of the other negative partitions;
(F) classifying the corrected partitions into categories according to the reason for correction;
(G) displaying the signal map, wherein all positive partitions are represented by a first indicator; and
(H) displaying the signal map, wherein each of the categories representing a reason for correction is represented by a second, third, fourth or further indicator;
wherein the graphical user interface further comprises at least two interactive tools, wherein in response to manipulation of at least one of the at least two interactive tools by a user, the method further comprises at least one of the following steps:
(I) adjusting the displayed signal map by excluding and/or including at least one category from being displayed; and /or
(J) choosing a category for one or more partitions and/or for one or more categories of at least one partition, thereby reclassifying said partitions as being of a chosen different category, and
   displaying the signal map, wherein the chosen different category is represented by an indicator specific for said category.

The method according to the first aspect is particularly advantageous for reviewing and manipulating data obtained by a biological assay, such as dPCR data, determined in a biological analysis system indicating the presence or absence of a target molecule, which preferably is a nucleic acid, in the analyzed biological sample. As disclosed herein, the method is in particular suitable for dPCR data processing revealing reliable results as a basis for data interpretation by the user.

According to a second aspect, a system for reviewing and manipulating of data obtained from signals determined by a biological analysis system is provided comprising
(a) at least one processor for performing the method according to the first aspect; and
(b) a memory encoded with instructions to perform the method according to the first aspect.

The system according to the second aspect may further comprise
(c) a display;
(d) a thermal cycler;
(e) an optical system;
(f) a storage device; and
(g) optionally an input device.

The system according to the second aspect is particularly suitable for detecting the presence or absence of a target molecule, such as a nucleic acid, in a biological sample by using a PCR-based approach, such as dPCR, and by performing the method for reviewing and manipulating the obtained data according to the first aspect.

According to a third aspect, a computer-readable storage medium is provided that is encoded with instructions, executable by a processor, for reviewing and manipulating of data, the instructions comprising instructions for performing the method according to the first aspect.

According to a fourth aspect, the present disclosure pertains to the use of a system according to the second aspect for performing the method according to the first aspect.

According to a fifth aspect, a use of the computer-readable storage medium according to the third aspect is provided for performing the method according to the first aspect.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

### BRIEF DESCRIPTION OF THE FIGURES

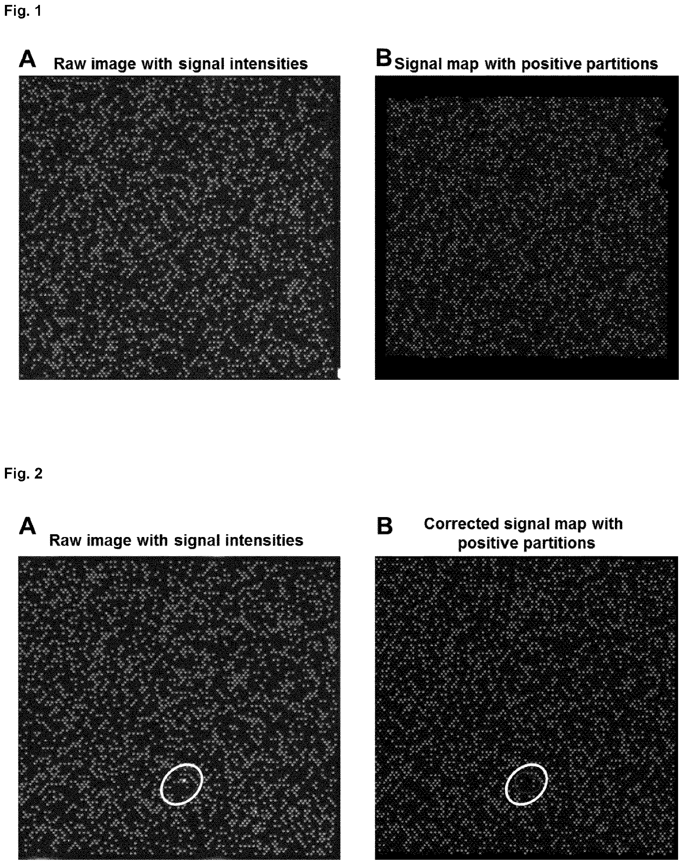

**Fig. 1**: (A) Raw image with fluorescence signal intensities according to their spatial location in one well of a multi-well plate as acquired by the camera. (B) Signal map representing positive partitions according to the spatial location of the underlying raw signal.
**Fig. 2**: **(A)** Raw image with fluorescence signal intensities according to their spatial location in one well of a multi-well plate as acquired by the camera. The encircled bright spot marks a bright signal caused by dust. **(B)** Signal map representing positive partitions according to the spatial location of the underlying raw signal and corrected for the false positive (i.e. a partition or signal that has to be corrected) signal caused by dust. The signal emitted from the dust is now excluded from the representation.

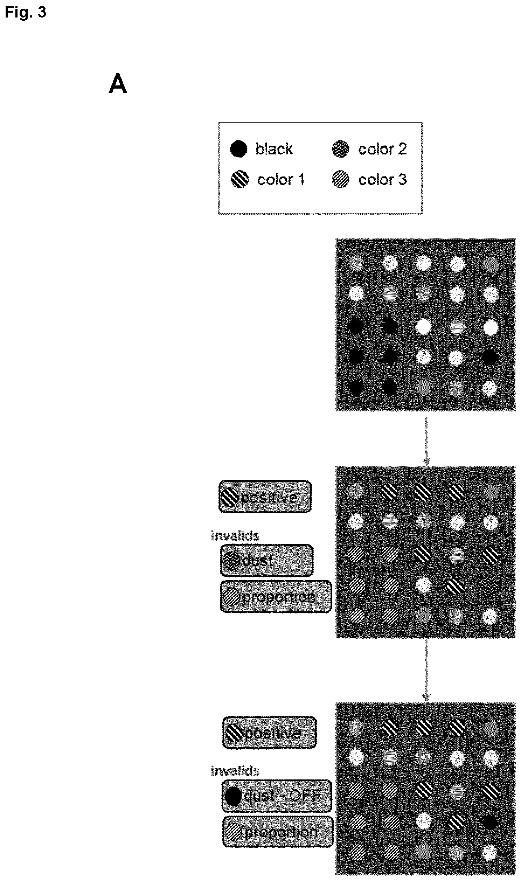

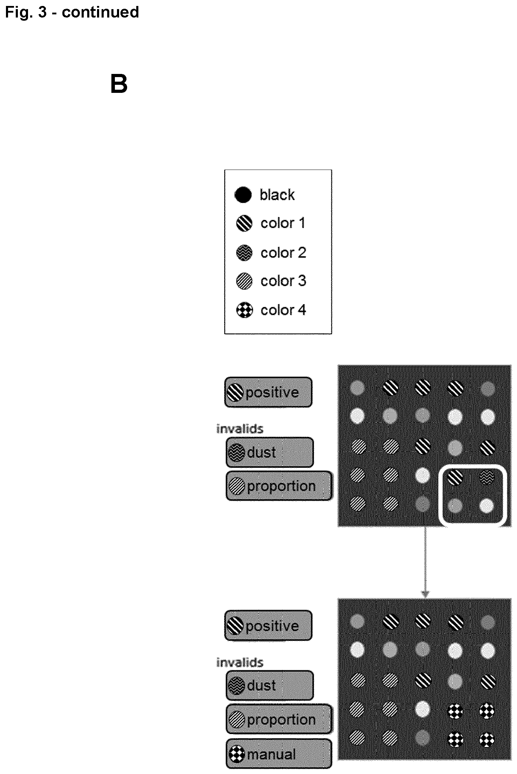

**Fig. 3****:** Exemplary workflows of the method for dPCR data reviewing and manipulating according to the invention. A signal map is displayed at a display comprising a graphical user interface (GUI). **(A)** The signal map showing pre-processed (corrected) dPCR data according to their spatial location is used as the basis (upper box). Corrected partitions may be excluded from representation while the valid partitions are represented according to their signal intensities. Alternatively, corrected partitions may be represented by a first indicator, such as a color (black). The positive partitions may be represented by a second indicator, such as a color (color 1 represented by circles with thick diagonals). Furthermore, corrected signals are visualized according to the reason for correction, such as "dust" or "proportion" (middle box). Each category of corrected signals is indicated by a different indicator, e.g. a color or a pattern. The user can manually select which categories shall be displayed by switching the individual categories off and on (bottom box). This is achieved by a first interactive tool such as a button for each category. Therefore, the first interactive tool provides the selection of all partitions of a category. **(B)** Single partitions or members from different categories can be manually selected by the user (upper box) and reclassified. Selection is achieved by a second interactive tool such as a box select tool that enables the user to select partitions of a certain part or area of the signal map independent of their categories as illustrated by the white box. The partitions of the selected area can be reclassified either to a different category already visualized or to a new category. For reclassification to a new category this new category can be assigned by the user, e.g. "manual" (bottom box). The results are updated accordingly.

### DETAILED DESCRIPTION OF THE INVENTION

The different aspects and embodiments of the invention disclosed herein make important contributions to the art as is also explained in the following.

### THE METHOD ACCORDING TO THE FIRST ASPECT

According to a first aspect, a method for reviewing and manipulating of data obtained from signals determined by a biological analysis system is provided, comprising the steps:
(A) receiving a plurality of detected signals from a plurality of partitions located at a region of a substrate subjected to a biological assay;
(B) choosing a value for a signal intensity threshold;
(C) classifying the partitions for which a signal intensity is detected above the signal intensity threshold as positive and/or the partitions for which a signal intensity is detected below the signal intensity threshold as negative;
(D) identifying partitions that have to be corrected, so that some or all of the partitions that have to be corrected are classified as negative and/or as invalid, so that the partitions that have to be corrected are corrected partitions and classifying all partitions classified as positive and negative as valid;
(E) displaying a graphical user interface comprising a signal map of the detected signals, wherein the signal map represents the valid partitions by their signal intensities, wherein the corrected negative partitions are represented according to the signal intensity of the other negative partitions;
(F) classifying the corrected partitions into categories according to the reason for correction;
(G) displaying the signal map, wherein all positive partitions are represented by a first indicator; and
(H) displaying the signal map, wherein each of the categories representing a reason for correction is represented by a second, third, fourth or further indicator;
wherein the graphical user interface further comprises at least two interactive tools, wherein in response to manipulation of at least one of the at least two interactive tools by a user, the method further comprises at least one of the following steps:
(I) adjusting the displayed signal map by excluding and/or including at least one category from being displayed; and /or
(J) choosing a category for one or more partitions and/or for one or more categories of at least one partition, thereby reclassifying said partitions as being of a chosen different category, and
   displaying the signal map, wherein the chosen different category is represented by an indicator specific for said category.

According to one embodiment, the negative partitions are represented by a further indicator in the signal map that differs from the indicators of steps (G), (H) and (J).

According to one embodiment, the indicator is selected from a color, a geometrical shape, a pattern, a symbol, an icon, a number, a character, a sign or any other mean suitable for distinction between the categories. According to an advantageous embodiment, the indicator is a color.

The terms "category" or "classification" may be used for the following categories or classifications: "positive partition", "negative partition", "valid partition", "invalid partition", "false negative partition" and "false positive partition". Further, the terms "category" or "classification" may be used for the categories or classifications that are related to a reason for correction.

The individual steps and preferred embodiments of the method according to the first aspect will now be described in detail.

### Steps (A) to (C)

In step (A) a plurality of detected signals are received from a plurality of partitions located at a region of a substrate subjected to a biological assay.

According to one embodiment, the detected signals are signals detected by an optical system. According to one embodiment, the signals are fluorescence signals emitted from at least one fluorophore. According to an advantageous embodiment, the detected signals are fluorescence signals emitted from at least one fluorophore and detected by an optical system. The optical system may be a camera suitable to detect the wavelength emitted from the at least one fluorophore used.

According to one embodiment, the wavelength of the fluorescence signals emitted from the at least one fluorophore is in the range of 500-700nm, preferably in a range selected from the group consisting of 510-550nm, 550-570nm, 580-610nm, 610-655nm, and 655-700nm. Each signal emitted from a fluorophore can be detected in an according channel of the optical system. According to an advantageous embodiment, two or more, three or more, four or more, or five or more fluorophores are used to detect the signals in two or more, three or more, four or more, or five or more different channels of the optical system. Several suitable fluorophores for the method according to the present invention are commercially available of which some are exemplary presented in the table below.

| **Channel** | **Excitation (nm)** | **Emission (nm)** | **Example fluorophores** |
|---|---|---|---|
| Green | 463-503 | 518-548 | FAM^{™}, EvaGreen^{®} |
| Yellow | 514-535 | 550-564 | HEX^{™}, VIC^{®}, JOE^{™} |
| Orange | 543-565 | 580-606 | TAMRA ^{™} |
| Red | 570-596 | 611-653 | ROX^{™}, Texas Red^{®} |
| Crimson | 590-640 | 654-692 | Cy5^{®} |

The substrate comprises hundreds, thousands, ten thousands or hundred thousand reaction sites, also termed partitions. Reaction sites or partitions as disclosed herein may include, but are not limited to, through-holes, wells, indentations, spots, cavities, sample retainment regions, and reaction chambers. According to one embodiment, the substrate comprises at least 20,000, at least 50,000 partitions, preferably at least 200,000 partitions, at least 600,000 partitions, at least 800,000 partitions. According to one embodiment, the substrate is a multi-well plate, such as an 8-well plate, a 24-well plate or a 96-well plate, comprising a plurality of partitions per well. According to an advantageous embodiment, the multi-well plate is selected from the group consisting of 24-well plate with 26,000 partitions per well, 24-well plate with 8,500 partitions per well, and 96-well plate with 8,500 partitions per well. According to one embodiment, the substrate is a microfluidic dPCR plate. According to one embodiment, a region of a substrate is represented by one or more wells of a multi-well plate. The skilled person can choose a suitable substrate for any biological assay, especially for dPCR. Such dPCR substrates are commercially available, e.g. the QIAcuity Nanoplates (QIAGEN, Hilden).

According to one embodiment, the biological assay performed in the biological analysis system is an amplification reaction. According to one embodiment, the amplification reaction has one or more of the following characteristics (i) it is a polymerase chain reaction (PCR); (ii) it is a digital polymerase chain reaction (dPCR); (iii) it is a reverse transcription amplification reaction; (iv) it is a reverse transcription PCR (RT-PCR); (v) it is an isothermal amplification reaction; (vi) it is a quantitative PCR; (vii) it is a quantitative reverse transcription PCR; (viii) it is an allele-specific PCR; (ix) it is an asymmetric PCR; (x) it is a ligation-mediated PCR; (xi) it is a multiplex PCR; (xii) it is a nested PCR; (xiii) it is a bridge PCR. According to an advantageous embodiment, the biological assay is a digital PCR or digital reverse-transcription PCR. The term "dPCR" as used herein refers to both digital PCR and digital RT-PCR. The dPCR system splits the sample to be analyzed into hundreds, thousands, ten thousands or hundred thousands subsamples. For instance, this is achieved in a microfluidic nanoplate comprising hundreds, thousands, ten thousands or hundred thousands reaction sites, in a microfluidic chip comprising hundreds, thousands, ten thousands or hundred thousands reaction sites (chip-based dPCR) or by splitting the sample into hundreds, thousands, ten thousands or hundred thousands droplets (droplet-based dPCR). Several digital (RT-)PCR systems are commercially available, such as the QIAcuity system (QIAGEN, Hilden).

In embodiments, the biological sample may comprise one or more types of biological targets, including DNA sequences (including cell-free DNA), RNA sequences, genes, oligonucleotides, molecules, proteins, biomarkers, cells (e.g., circulating tumor cells), or any other suitable target biomolecule.

The biological sample may be obtained from any biological source. According to one embodiment, the biological sample has one or more of the following characteristics:
(i) it is a bodily sample;
(ii) it is a human sample;
(iii) it is a pathogenic sample, such as a bacterium sample or a virus sample;
(iv) it is an animal sample;
(v) it is a cell culture sample;
(vi) it is a tissue sample;
(vii) it is an environment sample;
(viii) it is a wastewater sample; and/or
(ix) it is a plant sample.

A bodily sample may be a liquid body sample, such as blood, serum, plasma, or saliva, or a solid body sample, such as a biopsy from any tissue. Furthermore, the biological sample may be a swab including nasal swabs, oral swabs, nasopharyngeal swabs and oropharyngeal swabs. The sample may be obtained from a human subject suspected to be infected with a pathogen or to have a genetic disorder. Furthermore, the sample may comprise human material, such as human cells or cell components, e.g. human proteins or human nucleic acids.

According to one embodiment, the biological sample comprises nucleic acids. The nucleic acids may comprise or substantially consist of DNA, including genomic DNA, human DNA, pathogenic DNA, viral DNA, environmental DNA (eDNA). Alternatively, the nucleic acids may comprise or substantially consist of RNA, including mRNA, miRNA, siRNA, IncRNA, human RNA, pathogenic RNA, viral RNA.

According to one embodiment, the signals detected for the valid partitions are based on the presence or absence of at least one target nucleic acid in the biological sample analyzed in the biological analysis system.

According to one embodiment, in each partition a PCR is performed leading to amplification of the at least one target nucleic acid if present in the biological sample. If an amplification reaction occurs, a signal, such as a fluorescence signal, can be detected by the optical system in the respective partition(s) indicating the presence of the at least one target nucleic acid in the respective partition(s). In case no amplification reaction occurs no signal is detected by the optical system indicating that the at least one target nucleic acid is absent in the respective partition(s).

According to one embodiment, the at least one target nucleic acid has one or more of the following characteristics:
(i) it selected from RNA and/or DNA;
(ii) it is derived from a human;
(iii) it is derived from a pathogen, wherein the pathogen is selected from the group consisting of a virus, a bacterium, a protozoan, a viroid and a fungus; and/or
(iv) it is selected from viral RNA and/or viral DNA.

According to one embodiment, the target nucleic acid is derived from a RNA virus. According to one embodiment, the target nucleic acid is derived from a severe acute respiratory syndrome-related coronavirus, preferably severe acute respiratory syndrome coronavirus 2 (SARS-CoV-2), severe acute respiratory syndrome coronavirus (SARS-CoV or SARS-CoV-1) or Middle East Respiratory Syndrome (MERS), optionally wherein the target nucleic acid is derived from SARS-CoV-2, optionally wherein the one or more target nucleic acid sequences are selected from the SARS-CoV-2 genes N, N1, N2, RdRP, E and Orf1a/b.

According to one embodiment, the biological sample comprises at least one target nucleic acid associated with a genetic disorder. Any genetic disorder may be detected due to the presence or absence of the target nucleic acid associated with a genetic disorder, including cancer mutations, metabolic disorders, single-gene disorders, x-linked disorders, y-linked disorders, and chromosomal disorders.

According to one embodiment, the biological sample comprises at least one target nucleic acid associated with genome edits. Gene editing can be caused by insertion, deletion, modification or replacement of DNA in a genome. Several genetic engineering techniques are available, such as gene editing with Zinc finger nucleases or with CRISPR (Clustered Regularly Interspaced Short Palindromic Repeats) approaches.

In step (B) a value for a signal intensity threshold is chosen. The signal intensity may be given in Real Fluorescence Unit (RFU). According to one embodiment, the signal intensity threshold is generated according to the distribution of signal intensities from all signals detected from a plurality of partitions located at a region of a substrate subjected to a biological assay.

In step (C) the partitions for which a signal intensity is detected above the signal intensity threshold are classified as positive and/or the partitions for which a signal intensity is detected below the signal intensity threshold are classified as negative. The signal intensity threshold may be manually adjusted by the user in response to manipulation of an interactive tool, thereby changing the number of partitions classified as positive and negative accordingly.

### Steps (D) to (H)

In step (D) partitions that have to be corrected are identified, especially automatically. According to one embodiment, step (D) further comprises classifying some or all of the partitions that have to be corrected as negative and/or as invalid. Furthermore, step (D) comprises classifying all partitions classified as positive and negative as valid. In some embodiments, partitions that have to be corrected refer to partitions in which signals are detected that do not fulfill the characteristics of a signal representing a successful amplification reaction. In further embodiments, partitions may be assigned as "to be corrected" when they lie within an area of the region of a substrate representing an unequal distribution of positive partitions compared to all areas of that region of a substrate. Identification of partitions that have to be corrected may be based on several different reasons for correction. For instance, possible reasons for determining a signal that have to be corrected may be dust or other particles, scratches or other interfering factors that emit a signal that is detected by the optical system, an unequal distribution of positive partitions, statistically improbable clustering of positive partitions or any other erroneous event.

Thus, the term "partition that has to be corrected" encompasses a partition that gives reason for a correction. The reason for correction may be caused by the intensity signal for the partition itself or may be caused by the intensity signals of adjacent partitions and/or partitions that form a line or region with the considered partition. If a partition has been identified as a partition that has to be corrected and the partition is corrected, i.e. "(re)-classified", the partition is a corrected partition.

In embodiments, for the identification of partitions that have to be corrected caused by dust or other particles, scratches or any other interfering factors, different signal characteristics are determined. The identification of false positive or other partitions that have to be corrected, respectively, caused by these interfering factors can be based on the following reasons:
- shape: mismatch between shape of the dust/particle/scratch to the shape of a positive partition due to a successful amplification reaction including luminance distribution;
- position: mismatch between x- and y-coordinates in the image since partitions are regularly arranged in a grid;
- size: number of bright pixels that do not follow the regular luminance distribution of a positive partition received from a successful amplification reaction;
- luminance/clustering (only executed up to a certain threshold of number of positives): images with only a few positive partitions in which certain areas with many positive partitions occur due to diffuse dust.

In further embodiments, for the identification of partitions that have to be corrected caused by an unequal distribution of positive partitions, first, the overall number of positive partitions in the image is counted. In case the number of positive partitions reaches a certain value the following instructions are executed. The image is divided into smaller areas, such as squares, rectangles, rhombus, circles, ellipsis or any other mean suitable for image division, and then the number of positive partitions per area is counted. In case one of the areas shows too many or too few positive partitions, the partitions in this area are classified as partitions that have to be corrected.

In further embodiments, the identification of statistically improbable clustering of positives (e.g., in a row or column) is executed without violating the unequal distribution of positives. Based on the number of positive to negative partitions a probability calculation according to Poisson is performed. If there is an improbable cluster, all positive partitions except of a certain number of partitions, such as one, two, three or more partitions depending on the total number of partitions that have to be corrected identified, are classified as false positive, i.e. as partitions that have to be corrected.

Subsequently to the identification of partitions that have to be corrected, some or all of the partitions that have to be corrected are classified as negative and/or as invalid, so that the partitions that have to be corrected are corrected partitions. Furthermore, all partitions that are classified as positive or negative are further classified as valid. Negative partitions and positive partitions, i.e. valid partitions, are included in the final target nucleic acid quantification while invalid partitions are excluded from the final target nucleic acid quantification. Nevertheless, the invalid partitions are further included in the data reviewing and manipulation process prior to the final target nucleic acid quantification.

In step (E) a graphical user interface is displayed comprising a signal map of the detected signals. A "signal map" as described herein refers to the visualization of the spatial distribution of detected signals in the partitions and/or of the categories of the partitions in a x,y-coordinate. In step (E) the signal map represents the valid partitions by their signal intensities. Valid partitions are all positive and negative partitions, including corrected negative partitions and corrected positive partitions. Corrected negative partitions are represented in the signal map according to a value dependent on the signal intensity of the other negative partitions represented in the signal map. Corrected negative partitions can be represented by the average value of the other negative partitions represented in the signal map. Corrected negative partitions can also be represented by a value which is correlated to the signal intensity threshold which in turn depends on the value of the negative partitions. Corrected positive partitions are represented according to their signal intensity. Such a signal map with the raw signal or pre-processed signal, i.e. corrected signal, of each partition is shown in the examples.

In step (F) the corrected partitions are classified into categories according to the reason for correction. As explained above, several reasons for correction may cause a partition to be considered as a partition that has to be corrected, such as dust or other particles, scratches, other interfering factors that emit a signal, unequal distribution of positive partitions, and statistically improbable clustering of positive partitions. The skilled person is aware of any erroneous events that may cause false positive or other signals that may have to be corrected.

In step (G) the signal map is displayed in which all positive partitions are represented by a first indicator. As described above an indicator may be a color, a geometrical shape, a pattern, a symbol, an icon, a number, a character, a sign and/or any other mean suitable for distinction between the categories. As demonstrated in the examples, it is particularly advantageous to use a color as an indicator for visualizing and differentiating between different categories.

In step (H) the signal map is displayed in which each of the categories representing a reason for correction is represented by a second, a third, a fourth or further indicator, especially according to the number of categories displayed. According to one embodiment, the steps (G) and (H) are performed simultaneously. As demonstrated in the examples, the signal map may represent positive partitions by a first color, corrected partitions caused by dust by a second color and corrected partitions caused by unequal distribution by a third color.

### Steps (I) and (J)

The method according to the first aspect further allows the user to manipulate the processed data. Therefore, the graphical user interface further comprises at least two interactive tools.

According to one embodiment, in response to manipulation of at least one of the at least two interactive tools by a user, the method further comprises step (I) in which the signal map is adjusted by excluding and/or including at least one category from being displayed. As demonstrated in the examples, such an interactive tool may be a button for each category, a drop down menu showing all categories, a free hand selection tool by which one member of a certain category on the signal map is selected and thereby all partitions of that category are selected or any other suitable interactive tool may be used to select a certain category and thus all partitions of that selected category. According to one embodiment, the instruction in response to manipulation of this interactive tool is received by the system and the indicators for each partition of the selected category represented in the signal map are visualized accordingly. Alternatively, the raw signal or pre-processed data, such as exclusion of corrected partitions from representation as explained above, is visualized.

According to one embodiment, in response to manipulation of the other at least one of the at least two interactive tools by a user, the method further comprises step (J) in which a category for one or more partitions and/or for one or more categories of at least one partition is chosen, thereby reclassifying said partitions as being of a chosen different category and in which the signal map is displayed, wherein the chosen different category is represented by an indicator specific for said category. As demonstrated in the examples, such an interactive tool may be a box select tool to select a part or area of the signal map comprising one or more partitions or a free hand selection tool by selecting single partitions. Any tool suitable to select single partitions or a group of partitions independent of the respective category may be used. According to one embodiment, the selected partition(s) can be reclassified to a different category already displayed and the signal map is updated accordingly. According to an alternative embodiment, the selected partition(s) can be reclassified to a new category assigned by the user and indicating a different reason for correction. According to one embodiment, the graphical user interface may provide a box for manually entering the denotation of the new category. According to an advantageous embodiment, this new category is displayed by a different indicator, optionally selected by the user, and the signal map is updated accordingly. According to one embodiment, a suggestion is provided, which partitions may also be classified into a different category since the criteria for a different category may also be met. Such a suggestion may be displayed by a pop up message when the cursor is moved on a certain partition or by any other suitable mean to indicate that a certain partition may also be classified into a different category.

According to one embodiment, steps (I) and (J) are carried out one after the other.

### FURTHER EMBODIMENTS OF THE METHOD ACCORDING TO THE FIRST ASPECT

According to one embodiment, displaying further comprises displaying along with the signal map a multi-dimensional scatter plot of data points, representative of the signal emission intensity detected in the different channels. The signal intensity may be given in RFU. According to one embodiment, displaying further comprises displaying along with the signal map a one-dimensional scatter plot of data points, wherein the analyzed partition is plotted on the x-axis and the signal intensity is plotted on the y-axis. According to one embodiment, displaying further comprises displaying along with the signal map at least one two-dimensional scatter plot of data points representative of the signal emission intensity detected in two or more different channels, wherein the signal intensities detected in two different channels are plotted against each other. The degree of the dimension of the multi-dimensional scatter plot may be according to the number of fluorophores with different emission wavelengths used. Suitable fluorophores and their emission wavelengths are described elsewhere herein. According to this embodiment it is possible to obtain clusters of partitions emitting similar signal characteristics. For instance, in a two-dimensional scatter plot the signal intensities detected in two different channels due to the use of two different fluorophores in the amplification reaction are plotted against each other. Partitions for which a signal with a high intensity is detected in both channels may represent one cluster, partitions for which a signal with a high intensity is detected in one of the channels cluster together and partitions for which a signal with a low intensity or no signal is detected in both channels cluster together. This multi-dimensional scatter plot allows for further data processing. For example, a cluster analysis and/or principal component analysis may be performed to identify clusters of partitions with similar signal characteristics.

According to one embodiment, displaying further comprises displaying along with the signal map and optionally with the multi-dimensional scatter plot a histogram representation, wherein in the histogram the signal intensity is plotted on the x-axis and the number of partitions emitting a signal intensity is plotted on the y-axis. The signal intensity may be given in RFU. The histogram representation allows for visualization and reviewing of the number and distribution of positive and negative partitions in the region of a substrate. According to one embodiment, the histogram representation includes a representation of the signal intensity threshold, such as by a vertical line.

According to one embodiment, the signal intensity threshold can be adjusted by the user in response to manipulation of at least one graphical interactive tool. Such a tool may be included into the histogram representation, such as a slider or a box for entering a value for the signal intensity threshold. According to one embodiment, in response to manipulation of at least one graphical interactive tool for adjusting the signal intensity threshold the classifying and displaying steps are adjusted accordingly. By adjusting the signal intensity threshold the classification of positive and negative partitions may change accordingly as well as the identification of partitions that have to be corrected. Accordingly, also the classification of the corrected partitions into categories representing the reasons for correction may be changed and, consequently, also the signal map representation.

### THE SYSTEM ACCORDING TO THE SECOND ASPECT

According to the second aspect, a system for reviewing and manipulating of data obtained from signals determined by a biological analysis system is provided, the system comprising:
(a) at least one processor for performing the method according to the first aspect; and
(b) a memory encoded with instructions to perform the method according to first aspect.

According to one embodiment, the memory is a dynamic memory, preferably a random access memory (RAM). Such a RAM may have 4 GB, 8 GB, 16 GB, 32 GB or 64 GB, preferably the RAM has at least 16 GB. According to one embodiment, the memory is a static storage device, such as a read only memory (ROM).

According to one embodiment, the system further comprises
(c) a display;
(d) a thermal cycler;
(e) an optical system;
(f) a storage device; and
(g) optionally an input device.

According to one embodiment, the display comprises an input device. According to one embodiment, the display is a touchscreen. An input device may be an alphanumeric and other keys, which is coupled to bus for communicating information and command selections to processor, a cursor control, such as a mouse, a trackball or cursor direction keys for communicating information and command selections to processor and for controlling cursor movement on display.

According to one embodiment, the components (a) to (b) or (a) to (g) are connected to communicate and process information. The system may include bus or other communication mechanism for communicating information, and processor coupled with bus for processing information.

The system provides data processing, such as dPCR data processing. Data processing according to the method of the first aspect is provided by the system in response to processor executing one or more sequences of one or more instructions to perform the method according to the first aspect contained in memory. Such instructions may be read into memory from another computer-readable medium, such as storage device.

### COMPUTER-READABLE STORAGE MEDIUM ACCORDING TO THE THIRD ASPECT

In a third aspect, the present invention relates to a computer-readable storage medium encoded with instructions, executable by a processor, for reviewing and manipulating of data, the instructions comprising instructions for performing the method according to the first aspect.

The term "computer-readable medium" as used herein generally refers to any media that is involved in providing one or more sequences or one or more instructions for performing the method of the first aspect to processor for execution enabling the system to perform features or functions of embodiments of the present invention.

According to one embodiment, the computer-readable storage medium is selected from the group consisting of non-volatile media, volatile media, and transmission media. According to one embodiment, the computer-readable storage medium is a USB (Universal Serial Bus) flash drive. According to one embodiment, the computer-readable storage medium is a compact disc read-only memory (CD-ROM). The computer-readable storage medium can be read by a device which is connected to the processor by direct hardware or the computer-readable storage medium may be stored remote to the device, especially in a cloud to which the processor can have access.

### USE ACCORDING TO THE FOURTH AND FIFTH ASPECT

According to a fourth aspect, the use of the system according to the second aspect is provided to perform the method for data reviewing and manipulating according to the method of the first aspect.

According to a fifth aspect, the use of the computer-readable storage medium of the third aspect is provided to perform the method for data reviewing and manipulating according to the method of the first aspect.

### EXAMPLES

It should be understood that the following examples are for illustrative purpose only and are not to be construed as limiting this invention in any manner.

The following examples demonstrate the superior performance of the method for visualization of dPCR data according to the present invention.

### Abbreviations:

- dPCR: digital polymerase chain reaction
- GUI: graphical user interface
- PCR: polymerase chain reaction
- RFU: Real Fluorescence Unit

The following examples are preferably performed with the QIAcuity system (QIAGEN, Hilden) and corresponding kits. With respect to sample preparation, consumables, buffers and reagents, thermocycling, imaging, and further devices required reference is made to the QIAcuity User Manual (QIAGEN, Hilden, 03/2021 and Extension 12/2020). The QIAcuity Software Suite (QIAGEN, Hilden) provides a signal map for each well as a basis for the method of the invention.

For performing the method of the present invention, the following system requirements are recommended according to the QIAcuity User Manual (QIAGEN, Hilden, 03/2021):
- processor: x64 compatible processor with 4 physical cores and 2.5 GHz;
- memory: 16 GB RAM;
- storage device: at least 500 GB; and
- graphics: at least 1920x1080 pixels.

### Example 1: Automatic exclusion of invalid signals from dPCR data visualization

In dPCR, each well is divided into lots of sub-samples, i.e. partitions. For each of those sub-samples an end point PCR is executed and a signal is measured in a certain physical position. **Fig. 1A** shows an image representing the raw signals of the partitions of one 9mm x 9mm well acquired by a camera. Each partition is represented according to its respective spatial location and its signal intensity (RFU, Real Fluorescence Unit). The signal intensity (RFU) depends on the amplification reaction meaning that partitions where an amplification reaction occurred are represented by a bright signal (white spots in **Fig. 1A****)** while for partitions where no amplification reaction occurred no signal is detected. In partitions, in which an amplification reaction occurred but in which a low number of target nucleic acid molecules are present, a signal may still be detected but it may have a lower intensity. According to the specific characteristics of the signal, the partition is classified.

Visualization of the position of the partition together with its classification or category, respectively, in a signal map provides an overall context for data interpretation.

### Automatic classification and visualization of positive partitions

The partitions are automatically classified as positive/negative with respect to the signal intensity (i.e. amplification of the target nucleic acid) based on a signal intensity threshold. This threshold is calculated based on the distribution of all signal intensities detected in the respective well. The signal intensity threshold can also be set or adjusted manually by the user, e.g. in case a histogram of all detected signal intensities is presented on the graphical user interface (GUI) an interactive tool may be included, such as a slider or a box for entering a value. Partitions exhibiting a signal intensity value (RFU) above the signal intensity threshold are classified as positive while partitions exhibiting a signal intensity value below this threshold are classified as negative. The positive partitions can then be visualized in the signal map by a first indicator, such as a color, a geometrical shape, a pattern, a symbol, an icon, a number, a character or any other suitable indicator (bright spots in **Fig. 1B****).**

### Automatic identification and correction of partitions that have to be corrected

In addition, the signals detected for certain partitions or areas of the well may be considered to be incorrect, which means that the signals detected in these partitions do not fulfill the characteristics of a signal representing a successful amplification reaction or other erroneous events occurred leading to an incorrect assignment of the respective partitions. For instance, possible reasons for correction may be dust or other particles emitting a signal, an unequal distribution of positive partitions, statistically improbable clustering of positive partitions or any other erroneous event.

For the identification of partitions that have to be corrected caused by dust or other particles and scratches, different signal characteristics are determined and then identified as partitions that have to be corrected accordingly (see encircled dust in **Fig. 2A****).** The identification of dust can be based on the following reasons:
- shape: mismatch between shape of the dust/particle/scratch to the shape of positive partition including luminance distribution;
- position: mismatch between x- and y-coordinates in the image since partitions are regularly arranged in a grid;
- size: number of bright pixels that do not follow the regular luminance distribution of a positive partition;
- luminance/clustering (only executed up to a certain threshold of number of positives): images with only a few positive partitions in which certain areas with many positive partitions occur due to diffuse dust.

For the identification of partitions that have to be corrected caused by an unequal distribution of positive partitions, first, the overall number of positive partitions in the image is counted. In case the number of positive partitions reaches a certain value the following instructions are executed. The overall image is divided into smaller areas, such as squares, rectangles, rhombus, circles, ellipsis or any other mean suitable for image division, and then the number of positive partitions per area is counted. In case one of the areas shows too many or too few positive partitions, it is automatically classified as a partition that has to be corrected.

The identification of statistically improbable clustering of positives (e.g., in a row) is executed without violating the unequal distribution of positives. Based on the number of positive to negative partitions a probability calculation according to Poisson is performed. If there is an improbable cluster, all positive partitions except of a certain number of partitions, such as one, two, three or more partitions depending on the total number of partitions that have to be corrected identified, are classified as false positive, i.e. as corrected partition.

According to the reason of correction, some or all of the corrected partitions are either classified as negative, e.g. in case of improbable clustering of positives, and/or as invalid.

The signal map representing the positive partitions by a first indicator (e.g., color) is then corrected such that the corrected signals are excluded, i.e. not displayed anymore (see **Fig. 2B****).**

### Example 2: Visualization of categories of corrected partitions due to the reason for correction and manipulation by the user

Since the quantification of target nucleic acids is based on positive and negative partitions (i.e. valid partitions) indicating the presence or absence of the target nucleic acids, invalid partitions are excluded from the final target nucleic acid quantification. However, the corrected partitions, including invalid partitions, are included in the data reviewing and manipulating process to allow the user to include or exclude certain partitions in the analysis which is not provided by prior art methods.

The method according to the present invention expands the automatic visualization to not only distinguish between positive/negative and valid/invalid information, respectively, but also includes information on the reason for correction for each partition. Moreover, users can manually select which information shall be displayed together. Even further, users can manually change the automatic validity judgement, e.g. excluding certain partitions from the results or reclassify the visualized data.

**Fig. 3** illustrates exemplary workflows of the method according to the invention. These workflows can be advantageously implemented in the QIAcuity system (QIAGEN, Hilden). As a basis, a signal map for each well showing the partitions, optionally with the pre-processed or corrected data, is visualized as described above. In the exemplary workflow, the signal map represents valid signals according to the respective fluorescence signal intensity while invalid/corrected signals or partitions may automatically be identified and excluded from visualization or represented by a first indicator as described above (upper Box of **Fig. 3A****).**

### Automatic visualization of categories of corrected partitions

According to the method of the invention, several overlays represented by different indicators, such as colors, geometrical shapes, patterns, symbols, icons, numbers, characters or a similar representation suitable for distinction, visualize different categories of the corrected partitions due to the reason for correction.

Partitions that have to be corrected are automatically identified as described above. However, according to the present invention, the invalid/corrected partitions are not excluded from visualization as in prior art methods but visualized according to the respective reason for correction (e.g., dust/particles/scratch, unequal distribution of positive partitions, statistically improbable clustering of positive partitions, etc.). Each reason for correction is indicated by a certain indicator, such as a color, geometrical shape, pattern, symbol, icon, number, character or in a similar manner. **Fig. 3A** shows a signal map not only representing the positive partitions in the same color (color 1, circles with thick diagonals), but also the categories "dust" in color 2 (circles with undulating pattern) and "proportion" (for unequal distribution) in color 3 (circles with thin diagonals) (middle box, **Fig. 3A****).** Thus, the user receives the information on the reason for correction of certain partitions or certain areas.

### Manual selection of categories by the user

According to the present invention, the user can select which categories shall be displayed. This is achieved by an interactive tool present on the GUI allowing for user interaction. Such an interactive tool may be a button for each category as illustrated by the buttons in **Fig. 3****.** Alternatively or in addition, the user may directly select one member of a certain category on the signal map or by a drop down menu and thereby switching off or on the respective category. However, any other suitable interactive tool may be used to select a certain category.

The instruction in response to manipulation of this interactive tool is received by the system and the indicators (e.g., colors) presented in the signal map are updated accordingly. For instance, the user can switch off the visualization of the corrected partitions caused by "dust" (middle and bottom box in **Fig. 3A** represented by color 2). Furthermore, the category "dust" can be again switched on.

### Manual reclassification by the user

The method of the present invention further provides the user to select single partitions or members from different categories by a further interactive tool. Such an interactive tool may be a box select tool to select a part or area of the signal map (see upper box in **Fig. 3B****,** illustrated by the white box) or a free hand selection tool by selecting single partitions. Any tool suitable to select single partitions or a group of partitions independent of the respective category may be used. The system may provide a suggestion regarding partitions that may also fulfill the criteria of a different category. Such a suggestion may be displayed to the user by a pop up message or a similar mean.

The selected partition(s) can then be reclassified to a different category already displayed and the signal map is updated accordingly. Alternatively, the selected partition(s) can be reclassified to a new category assigned by the user and indicating a different reason for correction. Therefore, the GUI may provide a box for manually entering the denotation of the new category. This new category is displayed by a different indicator/color, optionally selected by the user, and the signal map is updated accordingly (bottom box in **Fig. 3B****;** category "manual" represented by color 4 (circles with checkerboard pattern)).

The method of the invention extends prior art methods for dPCR data visualization, reviewing and manipulation by the user and thereby provides improved and reliable dPCR data analysis, especially in tests for nucleic acids derived from viruses, bacteria, genetic disorders or any other target nucleic acids also in complex samples and samples containing minimal amounts of target nucleic acids.

## Claims

1. A method for reviewing and manipulating of data obtained from signals determined by a biological analysis system, comprising the steps:
(A) receiving a plurality of detected signals from a plurality of partitions located at a region of a substrate subjected to a biological assay;
(B) choosing a value for a signal intensity threshold;
(C) classifying the partitions for which a signal intensity is detected above the signal intensity threshold as positive and/or the partitions for which a signal intensity is detected below the signal intensity threshold as negative;
(D) identifying partitions as partitions that have to be corrected, so that some or all of the partitions that have to be corrected are classified as negative and/or as invalid, so that the partitions that have to be corrected are corrected partitions, and classifying all partitions classified as positive and negative as valid;
(E) displaying a graphical user interface comprising a signal map of the detected signals, wherein the signal map represents the valid partitions by their signal intensities, wherein the corrected negative partitions are represented according to a value dependent on the signal intensity of the other negative partitions represented in the signal map;
(F) classifying the corrected partitions into categories according to a reason for correction;
(G) displaying the signal map, wherein all positive partitions are represented by a first indicator; and
(H) displaying the signal map, wherein each of the categories representing a reason for correction is represented by a second, third, fourth or further indicator;
wherein the graphical user interface further comprises at least two interactive tools, wherein in response to manipulation of at least one of the at least two interactive tools by a user, the method further comprises at least one of the following steps:
(I) adjusting the displayed signal map by excluding and/or including at least one category from being displayed; and /or
(J) choosing a category for one or more partitions and/or for one or more categories of at least one partition, thereby reclassifying said partitions as being of a chosen different category, and
displaying the signal map, wherein the chosen different category is represented by an indicator specific for said category.

2. The method according to claim 1, wherein the signal map in (E) has one of the following characteristics:
(i) it represents the corrected partitions by a specific indicator that differs from the indicators of steps (G), (H) and (J); or
(ii) the corrected partitions are excluded from representation.

3. The method according to claim 1 or 2, wherein the negative partitions are represented by a further indicator in the signal map that differs from the indicators of steps (G), (H) and (J).

4. The method according to any one of claims 1 to 3, wherein the steps (G) and (H) are performed simultaneously.

5. The method according to any one of claims 1 to 4, wherein steps (I) and (J) are carried out one after the other.

6. The method according to any one of claims 1 to 5, wherein the indicator is selected from a color, a geometrical shape, a pattern, a symbol, an icon, a number, a character, a sign and/or any other mean suitable for distinction between the categories.

7. The method according to any one of claims 1 to 6, wherein the detected signals are fluorescence signals emitted from one or more, two or more, three or more, four or more or five or more fluorophores detected by an optical system.

8. The method according to any one of claims 1 to 7, wherein the signal intensity threshold is generated according to the distribution of signal intensities from all signals detected from a plurality of partitions located at a region of a substrate subjected to a biological assay.

9. The method according to any one of claims 1 to 8, wherein the signal intensity threshold can be adjusted by the user in response to manipulation of at least one graphical interactive tool.

10. The method according to claim 9, wherein in response to manipulation of at least one graphical interactive tool for adjusting the signal intensity threshold the classifying and displaying steps are adjusted accordingly.

11. The method according any one of claims 1 to 10, wherein the substrate has one or more of the following characteristics:
(i) it comprises at least 20,000, at least 50,000 partitions, preferably at least 200,000 partitions, at least 600,000 partitions, at least 800,000 partitions;
(ii) it is a multi-well plate comprising a plurality of partitions per well; and/or
(iii) it comprises at least one region, optionally wherein a region of a substrate is represented by one or more wells of a multi-well plate.

12. The method according to any one of claims 1 to 11, wherein the biological sample analyzed in the biological assay comprises nucleic acids.

13. The method according to any one of claims 1 to 12, wherein the signals detected for the valid partitions are based on the presence or absence of a target nucleic acid in the sample analyzed in the biological analysis system.

14. The method according to any one of claims 1 to 13, wherein the biological assay performed in the biological analysis system is an amplification reaction.

15. The method according to claim 14, wherein the amplification reaction has one or more of the following characteristics (i) it is a polymerase chain reaction (PCR); (ii) it is a digital polymerase chain reaction (dPCR); (iii) it is a reverse transcription amplification reaction; (iv) it is a reverse transcription PCR (RT-PCR); (v) it is an isothermal amplification reaction; (vi) it is a quantitative PCR; (vii) it is a quantitative reverse transcription PCR.

16. A system for reviewing and manipulating of data obtained from signals determined by a biological analysis system, the system comprising:
(a) at least one processor for performing the method according to any one of claims 1 to 15; and
(b) a memory encoded with instructions to perform the method according to any one of claims 1 to 15.

17. The system according to claim 16, wherein the system further comprises
(c) a display;
(d) a thermal cycler;
(e) an optical system;
(f) a storage device; and
(g) optionally an input device.

18. The system according to claim 16 or 17, wherein the components (a) to (b) or (a) to (g) are connected to communicate and process information.

19. A computer-readable storage medium encoded with instructions, executable by a processor, for reviewing and manipulating of data, the instructions comprising instructions for performing the method according to any one of claim 1 to 15.
